# EUROPEAN PATENT APPLICATION

(11) **EP 3 106 179 A1**
(43) Date of publication of application: **21.12.2016**
(21) Application number: 15748453.6
(22) Date of filing: 11.02.2015
(51) Int. Cl.: A61L 9/04, A61L 9/12

(54) **DEVICE FOR EVAPORATING VOLATILE SUBSTANCES**

(30) Priority: 12.02.2014 ES 201430186
(71) Applicant: Zobele España, S.A., 08019 Barcelona (ES)
(72) Inventor: MASO SABATÉ, Jordi, E-08019 Barcelona (ES); RUIZ BALLESTEROS, Julio, Cesar, E-08019 Barcelona (ES); MORHAIN, Cedric, E-08019 Barcelona (ES); MAYOR SANS, Fernando, E-08019 Barcelona (ES)
(74) Representative: Herrero & Asociados, S.L.
(86) International application number: PCT/ES2015/070087
(87) International publication number: WO 2015/121522

(57) **Abstract**

The device for evaporating volatile substances comprises a container (1) for a liquid that contains volatile substances and means (2) for evaporating the volatile substances. Said device is characterised in that said means for evaporating the volatile substances comprise two semipermeable membranes (2).

The device allows the optimisation of the evaporation of the volatile substances present in the liquid, doubling the evaporation surface thereof compared to conventional devices, and it increases the user's perception of the amount of liquid evaporated.

## Description

The present invention relates to a device for evaporating volatile substances, in particular a device for evaporating a fragrance or an active ingredient.

### Background of the invention

Devices for evaporating volatile substances that comprise of a container for liquids to evaporate fragrances or active ingredients are well known in the market.

In some cases, these devices consist of a container, usually made by thermoforming, which is coated with a semipermeable membrane. These devices are very often considered to have reduced effectiveness, which is linked to a lower evaporation rate.

One obvious way to improve the evaporation rate would be to increase the surface of the membrane; however, this implies a proportional increase in the size of the device, which is often not acceptable due to aesthetic reasons and cost.

Furthermore, attempts have been made to improve the evaporation surface by duplicating the number of containers of liquid provided with the corresponding membranes thereof. Evidently, two containers of liquid enable twice the amount to be evaporated compared to a single container of liquid.

This has been carried out in a very simple way, by introducing two separate containers in a support element, or in a more complex way, through a double container, that is, a single cartridge or replacement with two recipients, each one closed by a membrane.

When this double container is used, the container folds upon itself in order to occupy the least amount of space possible.

These devices have the following drawbacks.

Firstly, they require additional handling by the user. In the case of two containers, the user, who is accustomed to changing just one container, may not understand how it works. In addition, the user may mix two containers of different perfumes, which may be incompatible with respect to the mixture of the volatile substance created. Furthermore, if the two containers do not contain the same formula, they may have different durations, which may confuse the user.

Since the double containers can fold upon themselves, they have a very poor presentation, which is why they are usually hidden inside a decorative element. Furthermore, as the container must be folded, users must perform additional handling, which must be the reason why these containers are not usually sold as replacements. In addition, by being able to evaporate the substance through two separate containers, it is possible that due to internal influences (heat, airflow, etc.) one of the containers runs out of liquid before the other, which confuses the user.

Therefore, the need for a device for evaporating volatile substances is necessary.

### Description of the invention

The evaporation device of the invention resolves the aforementioned drawbacks and has other advantages which are described below.

The device for evaporating volatile substances according to the present invention comprises a container for a liquid that contains volatile substances and means for evaporating the volatile substances. Said device is characterised in that said means for evaporating the volatile substances comprise two semipermeable membranes.

Advantageously, said two semipermeable membranes are placed on opposite sides of the container.

According to a preferred embodiment, said container is defined in the central area of a frame, and said frame has a thickness greater than 2 millimetres, preferably greater than 4 millimetres.

Advantageously, said semipermeable membranes are placed on the largest faces of that frame.

If desired, each semipermeable membrane may be linked to a wick, preferably a paper wick.

In order to refill the container, the device for evaporating volatile substances according to the present invention further comprises at least one hole to connect said container to the outside.

The device for evaporating volatile substances according to the present invention, optimised the evaporation of volatile substances present in the liquid, doubling the evaporation surface thereof compared to conventional devices, and it increases the user's perception of the amount of liquid evaporated. In addition, it occupies less volume and does not confuse the user who uses it.

### Brief description of the drawings

For the purpose of helping to make the foregoing description more readily understandable, it is accompanied by a set of drawings that, schematically and by way of illustration and not limitation, represent an embodiment.

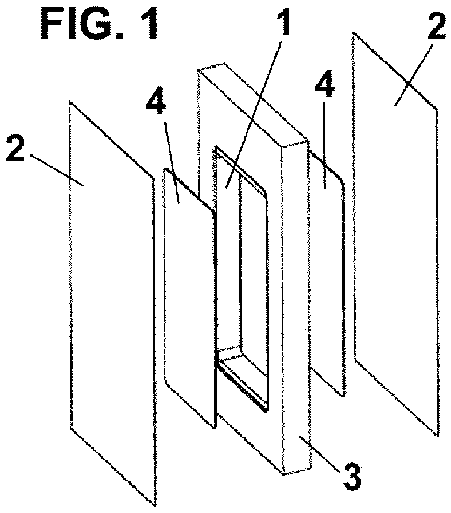

Figure 1 is a perspective view of the device for evaporating volatile substances according to the present invention in an exploded view.

### Description of a preferred embodiment

The device for evaporating volatile substances according to the present invention comprises a container 1 for a liquid that contains said volatile substances, such as fragrances or active ingredients.

According to the embodiment shown, said container 1 is defined in a frame 3, for example, with a rectangular shape, which has a thickness greater than 2 mm, and preferably greater than 4 mm.

In this way, the distance between the faces is that which defines the volume of the container 1 and, therefore, the capacity of the liquid with volatile substances and, as a result, the duration of the useful life of the device.

As shown in the figure, said frame 3 defines the two large faces, upon which the two semipermeable membranes 2 are placed. Evaporation of the volatile substances present in the liquid is carried out through said semipermeable membranes 2.

These semipermeable membranes 2 are welded or adhered to each large face of the frame 3. In this way, the liquid remains inside the container 1 between the frame 3 and the two semipermeable membranes 2.

The device must be activated before its first use, and this activation is preferably done by removing the aluminium layer (not shown in the figure) that is strongly adhered to each semipermeable membrane 2.

The frame 3 itself has added functions: it serves as a rigid element that enables the user to use it as a grip in order to remove the aluminium layer on the semipermeable membranes, and it also serves a support when the device functions as a passive evaporation element.

According to the embodiment shown, a wick of paper 4 may be placed on the inner portion of each semipermeable membrane 2; in other words, between the semipermeable membrane 2 and the container 1. In this way, the surface of the membrane is impregnated throughout the duration of the liquid contained.

If desired, the frame may comprise one or more holes 5. In the case of the embodiment shown, the frame 3 comprises two holes 5 placed on one side of said frame 3.

These holes 5 serve to fill the container 1 with liquid. Once the container 1 is full, the holes are closed, for example, with welded plastic caps.

As an alternative solution, the frame 3 can also incorporate some type of system that indicates the end of use of the liquid in the container 1. This end-of-use system may consist of, for example, an additional channel (not shown) that is connected to the container 1 in order to view the level of the remaining liquid.

If desired, the frame 3 can contain more than one container 1 to contain more than one liquid.

The evaporation device according to the present invention may function as a passive apparatus, in an evaporation apparatus using heat, with a fan, etc., and in a portable format as well as when plugged into the mains.

Despite the fact that reference has been made to a specific embodiment of the invention, it is evident for a person skilled in the art that numerous variations and changes may be made to the evaporation device described, and that all the aforementioned details may be substituted by other technically equivalent ones, without detracting from the scope of protection defined by the attached claims.

## Claims

1. A device for evaporating volatile substances comprising a container (1) for a liquid that contains volatile substances and means (2) for evaporating the volatile substances, **characterised in that** said means for evaporating the volatile substances comprise two semipermeable membranes (2).

2. The device for evaporating volatile substances according to claim 1, wherein the two semipermeable membranes (2) are placed on opposite sides of said container (1).

3. The device for evaporating volatile substances according to claim 1 or 2, wherein said container (1) is defined in the central area of a frame (3).

4. The device for evaporating volatile substances according to claim 3, wherein said frame (3) has a thickness greater than 2 millimetres.

5. The device for evaporating volatile substances according to claim 3 or 4, wherein said frame (3) has a thickness greater than 4 millimetres.

6. The device for evaporating volatile substances according to claims 2 and 3, wherein said semipermeable membranes (2) are placed on the large faces of said frame (3).

7. The device for evaporating volatile substances according to claim 1 or 2, wherein each semipermeable membrane (2) is linked to a wick (4).

8. The device for evaporating volatile substances according to any of the previous claims, which further comprises at least one hole (5) that connects said container (1) to the outside.
